# EUROPEAN PATENT SPECIFICATION

(11) **EP 1 496 359 B1**
(45) Date of publication and mention of the grant of the patent: **30.06.2010**
(21) Application number: 03715495.2
(22) Date of filing: 27.03.2003
(51) Int. Cl.: G01N 33/53, C12N 15/00, C12Q 1/68

(54) **SUBSTRATES FOR HYBRIDIZATION AND METHOD OF USING THE SAME**
SUBSTRATE ZUR HYBRIDISIERUNG UND VERFAHREN ZU DEREN VERWENDUNG
SUBSTRATS POUR L'HYBRIDATION ET SON PROCEDE D'UTILISATION

(30) Priority: 29.03.2002 JP 2002095132
(43) Date of publication of application: 12.01.2005
(73) Proprietor: Japan Science and Technology Agency, Kawaguchi-shi, Saitama 332-0012 (JP)
(72) Inventor: NAKAMURA, Fumio, Tokyo 136-0072 (JP); Hara, Masahiko, Wako-shi, Saitama 351-0198 (JP); Hayashi, Junko, Wako-shi, Saitama 351-0198 (JP)
(74) Representative: Gervasi, Gemma
(86) International application number: PCT/JP2003/003815
(87) International publication number: WO 2003/083473

(56) References cited:
- EP-A- 0 995 804
- EP-A- 1 279 434
- WO-A1-01/44503
- WO-A2-00/43539
- JP-A- 5 260 972
- JP-A- 2003 043 037
- US-A- 5 861 244

## Description

### Technical Field

The present invention relates to a hybridization substrate and a complementarity test method employing the same.

### Background Art

In genetic diagnosis and the specification of pathogenic microbes, or the detection of single nucleotide polymorphisms, a nucleic acid probe is employed to detect a single nucleic acid (target nucleic acid). The nucleic acid probe is mixed with a target nucleic acid and hybridization of the nucleic acid and the target nucleic acid is detected using, for example, a label such as fluorescence present on the nucleic acid probe.

DNA probes are primarily employed as the nucleic acid probe because they are readily synthesized with DNA synthesizers. Fluorescent probes are often employed because of the ease with which nucleic acid probes that have hybridized with the target nucleic acid can be detected using them. However, there are cases in which RI and the like are employed instead of fluorescent labels.

In recent years, DNA chips and DNA microarrays in which multiple nucleic acid probes have been attached to a substrate have entered practical use in detecting target nucleic acids.

US 5,861,244 describes a method for assaying sequences based on the ability of selected sequences to form triple strand structures.

EP 995804 provides for a polynucleotide analysis method based on probes attached on a substrate, where the probe is double-stranded.

EP 1279434 is a EPC 1973 54(3) document for the commonly designated states DE, FR, GB and NL. It describes a polynucleotide analysis method based on a double strand nucleotide sequence.

In the manufacturing of DNA chips and DNA microarrays, it is necessary to immobilize DNA on a substrate. DNA is immobilized, for example, by combining a thiol to single-stranded DNA to obtained thiolated single-stranded DNA, which is then immobilized, for example, on a metal substrate. However, DNA that has been immobilized by this method assumes a collapsed structure on the substrate. Thus, there are problems in that surface coverage rate and activity are extremely low.

By contrast, there are numerous reports of attempts to incorporate an alkyl chain between single-stranded DNA and a thiol to improve the surface coverage rate and activity (for example, see J. Am. Chem. Soc. 1998, 120, 9787-9792).

However, this procedure has proved difficult to implement because substantial time and cost are required to modify (incorporate a long alkyl chain into) the single-stranded DNA and the incorporation of a mixture onto the surface is required.

Accordingly, the object of the present invention is to provide a hybridization substrate upon the surface of which DNA strands are immobilized, and to provide a complementarity test method employing this substrate.

### Disclosure of the Invention

The present invention, solving the above-stated problems, is as given below.
(1) A hybridization substrate having on a substrate surface thereof a branching nucleic acid strand comprising a principal strand of nucleic acid at least one portion of which is attached to the surface of the substrate and at least one portion of which is single-stranded, and at least one strand of single-stranded nucleic acid for probe that is partially hybridized to at least one portion of the single-stranded portion of the principal nucleic acid and
   wherein the principal strand of nucleic acid comprises a portion that is attached to the substrate surface and comprises an end that is free of the substrate surface, with the portion attached to the substrate surface being double-stranded and the portion that is free of the substrate surface being single-stranded.
(3) The hybridization substrate according to (1) or (2) wherein the single-stranded portion of the principal strand of nucleic acid has at least one site in the form of a sequence for hybridizing the single-stranded nucleic acid for the probe.
(4) The hybridization substrate according to any of (1) to (3) wherein the single-stranded portion of the principal strand of nucleic acid has a probe area within, or on the end of, the portion that is free of the substrate surface.
(5) The hybridization substrate according to any of (1) to (4) wherein the single-stranded nucleic acid for the probe has at least a probe area and an area for hybridizing with the single-stranded portion of the principal strand of nucleic acid.
(6) A hybridization substrate having on a substrate surface thereof a branching nucleic acid strand comprising a principal strand of nucleic acid at least one portion of which is attached to the surface of the substrate and at least one portion of which is single-stranded; at least one accessory strand of nucleic acid that is single-stranded and partially hybridized to at least a portion of the single-stranded portion of the principal strand of nucleic acid; at least one strand of single-stranded nucleic acid for probe partially hybridized to at least one portion of the single-stranded portion of the principal strand of nucleic acid; and/or at least one strand of single-stranded nucleic acid for probe partially hybridized to at least a portion of the accessory strand of nucleic acid.
(7) The hybridization substrate according to (6) wherein the principal strand of nucleic acid comprises a portion that is attached to the substrate surface and has an end that is free of the substrate surface, with the portion attached to the substrate surface being double-stranded and the portion that is free of the substrate surface being single-stranded.
(8) The hybridization substrate according to (6) or (7) wherein the single-stranded portion of the principal strand of nucleic acid and/or the accessory strand of nucleic acid has at least one site in the form of a sequence for hybridizing a strand of single-stranded nucleic acid for the probe.
(9) The hybridization substrate according to any of (6) to (8) wherein the single-stranded portion of the principal strand of nucleic acid has a probe area within, or on the end of, the portion that is free of the substrate surface.
(10) The hybridization substrate according to any of (6) to (9) wherein the accessory strand of nucleic acid has a probe area on the end of a portion not hybridizing with the single-stranded portion of the principal strand of nucleic acid.
(11) The hybridization substrate according to any of (6) to (10) wherein the accessory strand of nucleic acid has one site in the form of a sequence for hybridizing with the single-stranded portion of the principal strand of nucleic acid and at least one site in the form of a sequence for hybridizing with the single-stranded nucleic acid for the probe.
(12) The hybridization substrate according to any of (1) to (11) wherein the single-stranded nucleic acid for the probe has at least a probe area and an area for hybridization with the single-stranded portion of the principal strand of nucleic acid and/or the accessory strand of nucleic acid.
(13) The hybridization substrate according to (12) wherein the single-stranded nucleic acid for the probe has a spacer area between the probe area and the hybridization area.
(14) The hybridization substrate according to any of (1) to (13) wherein the branching nucleic acid strand has multiple probe areas, with the nucleic acid sequence of the multiple probe areas being identical.
(15) The hybridization substrate according to any of (1) to (13) wherein the branching nucleic acid strand has multiple probe areas, with the nucleic acid sequence of the multiple probe areas being different.
(16) The hybridization substrate according to any of (1) to (15) wherein there are multiple spots formed from multiple branching nucleic acid strands.
(17) A method comprising bringing target nucleic acid into contact with a surface upon which has been immobilized the branching nucleic acid strand of the substrate according to any of (1) to (16) to test the complementarity of the target nucleic acid with the probe area of the branching nucleic acid strand.
(18) The method according to (17) wherein the contact of the target nucleic acid with the surface upon which is immobilized the branching nucleic acid strand is conducted in the presence of a divalent metal ion.
(19) The method according to (18) wherein the divalent metal ion is a magnesium ion.
(20) The method according to any of (17) to (19) wherein hybridization of the target nucleic acid and the probe area of the branching nucleic acid strand is detected by the surface plasmon resonance method or the crystal oscillator method.
(21) The method according of any of (17) to (19) wherein the target nucleic acid is labeled with a fluorescent label and hybridization with the probe area of the branching nucleic acid strand is detected by fluorescence.
(22) The method according to any of (17) to (21) for detecting target nucleic acid containing mismatched nucleic acids.

### Brief Description of the Figures

Fig. 1 is a drawing descriptive of the branching nucleic acid strands on a substrate of Mode 1 of the present invention.
Fig. 2 shows the scheme of formation of probe DNA (branching DNA), immobilization on the substrate, and hybridization in an embodiment.
Fig. 3 shows the results of *in situ* observation by surface plasmon resonance of immobilization on a gold substrate surface when 50:50 and 100:0 ratios of probe DNA (branching DNA) to 20mer/20merSH complex were employed, and immobilization on a gold substrate surface for a 50:50 ratio of 80merP and 20merSH complex, employed instead of probe DNA (branching DNA), to 20mer/20merSH complex.
Fig. 4 shows the results of *in situ* observation by surface plasmon resonance for hybridization of target DNA 5'-CTGTGTCGATCAGTTCTCCA-3' (20merM) and control DNA 5'-CTGTGTCAATCAGTTCTCCA-3' (20merS) differing in sequence by just one nucleic acid.

### Best Mode of Implementing the Invention

### (Substrate)

The hybridization substrate of the present invention is characterized by having on a substrate surface a branching nucleic acid strand comprising a principal strand of nucleic acid at least one portion of which is attached to the surface of the substrate and at least one portion of which is single-stranded, and at least one strand of single-stranded nucleic acid for probe that is partially hybridized to at least one portion of the single-stranded portion of the principal nucleic acid. That is, the branching nucleic acid strand is comprised of one principal strand of nucleic acid and one or more strands of single-stranded nucleic acid for probes. A portion of the single-stranded nucleic acid for probes is hybridized to the single-stranded portion of the principal strand of nucleic acid.

According to a further embodiment of the present invention the substrate is characterized by having on a substrate surface a branching nucleic acid strand comprising a principal strand of nucleic acid at least one portion of which is attached to the surface of the substrate and at least one portion of which is single-stranded; at least one accessory strand of nucleic acid that is single-stranded and partially hybridized to at least a portion of the single-stranded portion of the principal strand of nucleic acid; at least one strand of single-stranded nucleic acid for probe partially hybridized to at least one portion of the single-stranded portion of the principal strand of nucleic acid; and/or at least one strand of single-stranded nucleic acid used as a partially hybridized to at least a portion of the accessory strand of nucleic acid. That is, the branching nucleic acid strand comprises a principal strand of nucleic acid; one or more accessory strands of nucleic acid; and one or more strands of single-stranded nucleic acid for probes. A portion of the accessory strand of nucleic acid is hybridized to a single-stranded portion of the principal strand of nucleic acid, and a portion of the single-stranded nucleic acid for probes is hybridized to a single-stranded portion of the principal strand of nucleic acid or the accessory strand of nucleic acid.

Both the first and second substrates of the present invention have a principal strand of nucleic acid constituting a branching nucleic acid strand. At least a portion of the principal strand of nucleic acid is attached to the surface of the substrate, and at least a portion of this principal strand is single-stranded.

The principal strand of nucleic acid will be described below.

At least a portion of the principal strand of nucleic acid is attached to the substrate surface. Specifically, one end is attached to the substrate surface and the other end is free of the substrate surface as this is desirable from the perspective of ease of manufacturing.

In said principal strand of nucleic acid with one end attached to the substrate surface and the other end free of the substrate surface, the portion that is attached to the substrate surface is be double-stranded, and the portion that is free of the substrate surface is single-stranded. The principal strand of nucleic acid can be DNA, RNA, or PNA, for example. The single-stranded portion of the principal strand of nucleic acid has at least one site with a sequence for hybridizing with a strand of single-stranded nucleic acid used as a probe. The number of sites with sequences for hybridizing with strands of single-stranded nucleic acid for probes is not specifically limited, and may be suitably determined based on the desired number of probe areas on a single branching nucleic acid strand. Further, the number of sites with sequences for hybridizing with strands of single-stranded nucleic acid for probes may also be suitably determined by taking into account the number of nucleic acids (length) of the area being hybridized and the number of nucleic acids (length) of the principal strand of nucleic acid that can be prepared. The sites of sequences for hybridizing with single-stranded nucleic acid for probes are not limited so long as being complementary with the areas of hybridization with the single-stranded nucleic acid for probes. From the perspective of stability of hybridization, the number of nucleic acids is suitably from 10 to 30, 10 to 20 being desirable and 15 being preferred. However, this range is not given by way of limitation.

The single-stranded portion of the principal strand of nucleic acid may have a probe area on the end of, or part way along, the portion that is free of the substrate surface.

When one end of the principal strand of nucleic acid is attached to the substrate surface and the other end is free of the substrate surface, one or more probe areas can be present at the tips of the portion(s) that are free of the substrate surface.

The number of nucleic acids and the sequence of the probe area in the single-stranded portion of the principal strand of nucleic acid can be suitably determined based on the application of the substrate of the present invention.

The structure of the principal strand of nucleic acid and the method of immobilizing it on the substrate surface are not specifically limited. For example, it may be single-stranded DNA attached to an Affymetrics-type DNA microarray or single-stranded DNA attached to a Stanford-type DNA microarray. Branching DNA can be manufactured by hybridizing to the single strand of DNA attached to the solid substrate at least one single-stranded accessory strand of DNA having a nucleic acid sequence portion that is complementary to the immobilized single strand of DNA.

Alternatively, the principal strand of nucleic acid may be a DNA strand having one portion that is double-stranded and another portion that is single-stranded, and be attached to the substrate surface on the double-stranded side. In such a DNA strand, one strand may be longer than the other strand so that one portion is double-stranded and the remaining portion is single-stranded, with the single-stranded portion beginning at the end of the double-stranded portion and running to the end of the longer strand. The principal strand of nucleic acid need not be DNA; for example, either the double-stranded portion or the single-stranded portion may be single-stranded RNA. In the double-stranded portion, one of the strands making up the double-stranded portion may be RNA.

The principal strand of nucleic acid may also be a strand of DNA with double-stranded portions at each end and a single-stranded portion in the middle, with the double-stranded portions at both ends being secured to the substrate surface.

Neither the number of nucleic acids in double-stranded portions nor that in single-stranded portions is restricted or limited. However, the number of nucleic acids in double-stranded portions can be kept within a range of from 10 to 80 to ensure stability of the double-stranded portions, and the number of nucleic acids in single-stranded portions can be suitably selected, for example, within a range of from 20 to 90 in consideration of the number of single-stranded nucleic acid strands and accessory strands of nucleic acid for hybridization probes and the length of the hybridization areas. However, greater lengths than these can be manufactured and employed in the present invention.

In such a DNA strand, there are two single strands of different length. The shorter of the single strands of DNA has a nucleic acid sequence that complements the nucleic acid sequence of the longer of the single strands of DNA from one end of the longer strand. Thus, such a DNA strand can be manufactured by hybridizing the two single strands.

A DNA strand having the above-described double-stranded portion and single-stranded portion is secured to the substrate surface on the double-stranded portion side. Such a configuration positions the double-stranded portion closer to the substrate surface and the single-stranded portion of the DNA strand away from the substrate surface. This affords the advantages of providing a certain amount of space around the single-stranded portion, facilitating the use of the single-stranded portion as a hybridization sequence, and densely positioning the double-stranded portions close to the substrate surface, preventing the collapse of the DNA strands.

In the case of a metal substrate or metal-coated substrate, for example, the DNA strand can be secured to the metal surface of the substrate by means of sulfur atoms. Examples of metal substrates are gold, silver, chromium, gallium, nickel, and neodymium. Examples of metal-coated substrates are substrates of glass, mica, or the like coated with a metal such as gold, silver, chromium, gallium, nickel, or neodymium.

A detailed description of securing the DNA strands through sulfur atoms to the metal surface of the substrate is given in the method of manufacturing the substrate.

When the substrate is glass or silicon, for example, the DNA strand can be secured with a sulfur atom to the surface of the substrate. An example of a glass substrate is the common glass slide. An example of a silicon substrate is a silicon wafer.

A detailed description of securing the DNA strands with sulfur atoms to the surface of the substrate is given in the method of manufacturing the substrate.

In addition to the above-described DNA strand having a double-stranded portion and a single-stranded portion, entirely double-stranded DNA may be further secured to the surface of the substrate. Further securing entirely double-stranded DNA to the substrate surface affords the advantages of increasing the space around the single-stranded portions free of the substrate surface, thus facilitating the use of the single-stranded portions as hybridization sequences, and densely positioning double-stranded portions near the substrate surface, preventing the collapse of the DNA strands.

The substrate may be a metal substrate or a metal-coated substrate, for example. In such cases, in addition to a DNA strand having a double-stranded portion and a single-stranded portion, entirely double-stranded DNA may be secured through sulfur atoms to the metal surface of the substrate. The substrate may also be a glass or silicon substrate, in which case entirely double-stranded DNA may be secured through sulfur atoms to the substrate surface in addition to DNA strands having double-stranded portions and single-stranded portions.

When entirely double-stranded DNA is secured to the substrate surface in addition to DNA strands having double-stranded portions and single-stranded portions, the ratio of the number of secured DNA strands having double-stranded portions and single-stranded portions to the number of strands of entirely double-stranded DNA can be suitably determined in consideration of the density (tightness) of the single-stranded portions serving as hybridization sequences. For example, a range of from 99:1 to 1:99, desirably from 99:1 to 25:75, is suitable.

### (Method of manufacturing the substrate)

In the case of a metal or metal-coated substrate, for example, DNA strands having double-stranded portions and single-stranded portions with thiol groups present on the ends of the double-stranded portions are brought into contact with the metal surface of the substrate to secure the DNA strands to the metal surface and manufacture substrates having principal strands of nucleic acid in the form of DNA. Double-stranded DNA having a double-stranded portion and a single-stranded portion can be manufactured by hybridizing two single strands of differing length, the nucleic acid sequence of the shorter strand of DNA being complementary to the nucleic acid sequence from one end of the longer single strand of DNA. Then, for example, by introducing a thiol group at the 5' terminal of the shorter single strand of DNA and hybridizing it with a longer single strand of DNA having a sequence from its 3' terminal complementary to the sequence of the shorter single strand of DNA, it is possible to obtain a DNA strand having a double-stranded portion and a single-stranded portion with a thiol group on the end of the double-stranded portion. For example, the known C6 synthesis method can be employed to introduce a thiol group at the 5' end of single-stranded DNA. (For example, see Chemical and Biology Experimental Line 22, Mineo NIWA, "The Chemical Synthesis of DNA", pp. 38-43, Hirokawa Shoten.)

The hybridization of the shorter single-stranded DNA and longer single-stranded DNA having a complementary sequence on the 3' end side can also be suitably conducted under the usual conditions by the usual methods.

Anchoring of the DNA strands to the metal surface of the substrate is conducted by bringing the DNA strands having double-stranded portions and single-stranded portions with thiol groups on the ends of the double-stranded portions into contact with the metal surface. The securing of DNA strands having thiol groups to metal surfaces is described in J. Am. Chem. Soc. 1998, 120, 9787-9792, for example, and is a known method.

Further, a mixture of a prescribed ratio of DNA strands having double-stranded portions and single-stranded portions with thiol groups on the ends of the double-stranded portions and entirely double-stranded DNA having terminal thiol groups can be brought into contact with the metal surface of a metal or metal-coated substrate in the same manner as above to secure DNA strands having double-stranded portions and single-stranded portions and entirely double-stranded DNA to the metal surface in a prescribed ratio.

To manufacture a glass or silicon substrate, the surface of the substrate is treated with a heterobifunctional crosslinking agent and the treated surface is brought into contact with DNA strands having double-stranded portions and single-stranded portions with thiol groups on the ends of the double-stranded portions to secure the DNA strands to the surface. The DNA strands having double-stranded portions and single-stranded portions with thiol groups on the ends of the double-stranded portions can be manufactured by the method set forth above.

As set forth above, a thiol group is incorporated onto the end of the double-stranded portion of the DNA strand having a double-stranded portion and a single-stranded portion. Moreover, the thiol group may be incorporated onto the shorter strand or the longer strand of the DNA strand having a double-stranded portion and a single-stranded portion. However, the thiol group is desirably incorporated onto the shorter strand constituting only the double-stranded portion. This is advantageous in that the longer strand having a single-stranded portion contributing to hybridization can be employed with no other processing than hybridization with the shorter strand.

This anchoring method is described in Nucleic Acids Research, 1996, Vol. 24, No. 15, 3031-3039, for example.

The above-mentioned heterobifunctional crosslinking agent may be one or more crosslinking agents selected from the group consisting of: succinimidyl 4-[maleimidophenyl] butyrate (SMPB), m-maleimidobenzoyl-N-hydroxysuccinimide ester (MBS), succinimidyl 4-(maleimidoethyl)cyclohexane-1-carboxylate (SMCC), N-(y-maleimidobutoxy)succinimide ester (GMBS), m-maleimidopropionic acid-N-hydroxysuccinimide ester (MPS), and N-succinimidyl(4-iodoacetyl)aminobenzoate (SIAB).

A mixture comprising a prescribed ratio of DNA strands having double-stranded portions and single-stranded portions with thiol groups on the ends of the double-stranded portions and entirely double-stranded DNA having terminal thiol groups can be brought into contact with the surface of a glass or silicon substrate that has been surface treated with a heterobifunctional crosslinking agent to secure the two types of DNA strands to the surface in a prescribed ratio.

In the method of bringing DNA strands having double-stranded portions and single-stranded portions with thiol groups on the ends of the double-stranded portions, or a mixture of a prescribed ratio of DNA strands having double-stranded portions and single-stranded portions with thiol groups on the ends of the double-stranded portions and entirely double-stranded DNA having terminal thiol groups, into contact with the surface of the metal surface of the above-described substrate to secure the DNA strands to the metal surface, the DNA strands are desirably brought into contact with the metal surface in the presence of divalent metal ions. Conducting this method in the presence of divalent metal ions affords the advantages of increasing the stability of the double-stranded portions and stably aggregating the double-stranded portions resulting in securing stable existence on the substrate.

Examples of divalent metal ions are: magnesium ions, calcium ions, cobalt ions, barium ions, strontium ions, cadmium ions, zinc ions, and iron ions. The concentration of these divalent metal ions suitably falls within a range of from 1 to 1,000 mM.

Similarly, in the method of bringing DNA strands having double-stranded portions and single-stranded portions with thiol groups on the ends of the double-stranded portions, or a mixture of a prescribed ratio of DNA strands having double-stranded portions and single-stranded portions with thiol groups on the ends of the double-stranded portions and entirely double-stranded DNA having terminal thiol groups, into contact with the substrate surface treated with the above-stated heterobifunctional crosslinking agent to secure the DNA strands to the metal surface, the DNA strands are desirably brought into contact with the surface in the presence of divalent metal ions for the same reasons as above.

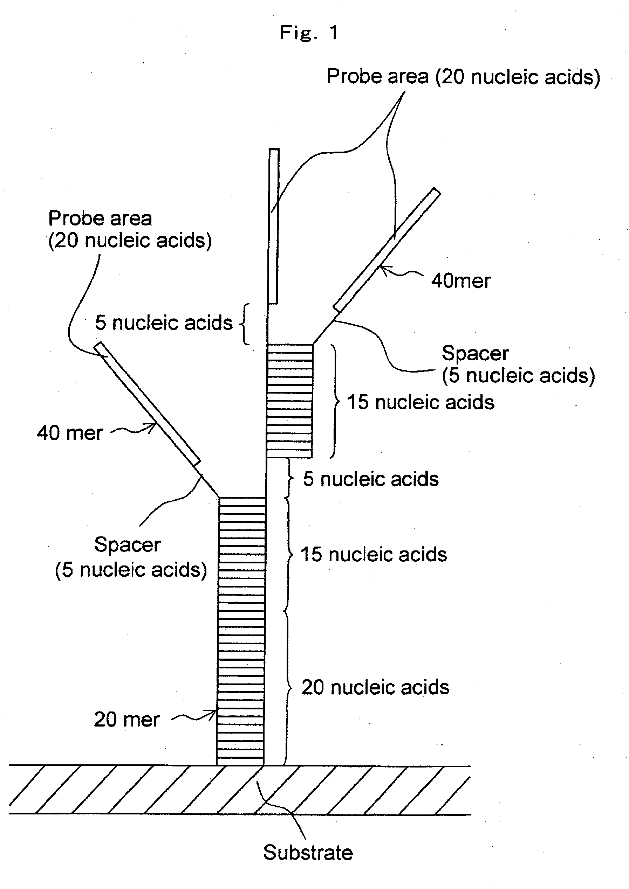

The first substrate of the present invention has a branching nucleic acid strand comprising at least one single-stranded nucleic acid for probe partially hybridized to at least one portion of the single-stranded principal nucleic acid. This will be described based on Fig. 1.

In the example given in Fig. 1, 80mer and 20mer single-stranded DNA hybridized at the end of the 80mer are employed as the principal strand of nucleic acid. The double-stranded portion of the 80mer and 20mer is secured to the substrate. The single-stranded portion of the principal strand of nucleic acid has 60 nucleic acids. These 60 nucleic acids have the following structure from the substrate side.

| | |
|---|---|
| 15 nucleic acids: | area for hybridizing to single-stranded nucleic acid for first probe |
| 5 nucleic acids: | spacer |
| 15 nucleic acids: | area for hybridizing to single-stranded nucleic acid for second probe |
| 5 nucleic acids: | spacer |
| 20 nucleic acids: | probe area |

The single-stranded nucleic acid for probe may be DNA, RNA, or PNA, for example. It comprises at least a probe area and an area of hybridization with the single-stranded portion of the principal strand of nucleic acid. A spacer area may be present between the probe area and the hybridization area. The portion of the sequence of the single-stranded nucleic acid for probe used for hybridization with the single-stranded portion of the principle strand of nucleic acid is not specifically limited other than it be complementary to the single-stranded portion of the principal strand of nucleic acid. From the perspective of hybridization stability, a number of nucleic acids of from 10 to 30 is suitable, 10-20 is desirable, and 15 is preferred. However, this range is not given by way of limitation.

The sequence and number of nucleic acids of the probe area may be suitably determined based on the application of the substrate of the present invention.

The single-stranded nucleic acid of first and second probes shown in Fig. 1 comprises 40 nucleic acids consisting of a 15-nucleic acid hybridization area, a 5-nucleic acid spacer, and a 20-nucleic acid probe area.

The number of single-stranded nucleic acid strands for probe used for hybridization on the single-stranded portion of the principal strand of nucleic acid can be suitably determined by adjusting the length of the single-stranded portion of the principal strand of nucleic acid and the length of the hybridization areas of the single-stranded nucleic acid used for the probes. From the perspective of facilitating the manufacturing of the principal strand of nucleic acid, the length of the principal strand of nucleic acid desirably falls within a range of from 50 to 200 nucleic acids.

However, this length may be increased as techniques for manufacturing nucleic acid improve.

In the second substrate of the present invention, there is at least one single-stranded accessory strand of nucleic acid partially hybridizing with at least a portion of the single-stranded portion of the principal strand of nucleic acid.

The accessory strand of nucleic acid may be DNA, RNA, or PNA, for example, and has an area for hybridization with the single-stranded portion of the principal strand of nucleic acid and an area for hybridization with the single-stranded nucleic acid for probe. Further, the accessory strand of nucleic acid may have a probe area on its tip on the side not hybridizing with the single-stranded portion of the principal strand of nucleic acid. From the perspective of facilitating the manufacturing of the accessory strand of nucleic acid, the length of the accessory strand of nucleic acid desirably falls within a range of from 50 to 200 nucleic acids.

However, this length may be increased as techniques for manufacturing nucleic acid improve.

In the branching nucleic acid strand present on the second substrate of the present invention, at least one strand of single-stranded nucleic acid for probe is partially hybridized on at least a portion of the accessory strand of nucleic acid. Further, at least one strand of single-stranded nucleic acid for probe may also be partially hybridized on at least a portion of the single-stranded portion of the principal strand of nucleic acid.

In the second substrate of the present invention, the number of strands of single-stranded nucleic acid for probes hybridized with a single branching nucleic acid strand can be increased, and when testing for complementarity, the signal intensity can be increased, by hybridizing strands of single-stranded nucleic acid for probes to the accessory strands of nucleic acid branching off of the principal strand of nucleic acid (and in some cases, to the principal strand of nucleic acid).

In the substrate of the present invention, when multiple probe areas are present on the branching nucleic acid strand, the sequence of the multiple probe areas may be identical to hybridize with a single target nucleic acid.

In the substrate of the present invention, when multiple probe areas are present on the branching nucleic acid strand, some or all of the sequences of the multiple probe areas may be different to permit a single branching nucleic acid strand to hybridize with multiple target nucleic acids having different sequences.

Further, the substrate of the present invention may consist of multiple spots on a single substrate surface, with multiple branching nucleic acid strands being formed at each spot. The size of a single spot and the number (density) of branching nucleic acid strands at a given spot can be suitably selected in consideration of the application of the substrate. Further, the number of spots formed on a substrate can also be suitably determined.

The substrate of the present invention can be manufactured by a number of methods.

Examples of methods of manufacturing the substrate of mode 1 are:
(1) Preparing a substrate with principal strands of nucleic acid secured on the substrate surface and hybridizing strands of single-stranded nucleic acid for probes with the principal strands of nucleic acid on the substrate.
(2) Preparing branching nucleic acid strands by hybridizing strands of single-stranded nucleic acid for probes with the principal strands of nucleic acid and securing the resulting branching nucleic acid strands to the substrate surface.

Examples of methods of manufacturing the substrate of mode 2 are:
(1) Preparing a substrate with principal strands of nucleic acid secured on the substrate surface, hybridizing accessory strands of nucleic acid with the principal strands of nucleic acid on the substrate, and hybridizing strands of single-stranded nucleic acid for probes with the accessory strands of nucleic acid (and when necessary, with the principal strands of nucleic acid).
(2) Hybridizing accessory strands of nucleic acid to the principal strands of nucleic acid to prepare branching nucleic acid strand precursors, securing these branching nucleic acid strand precursors to the substrate surface, and hybridizing strands of single-stranded nucleic acid for probes with the branching nucleic acid strand precursors (the portions consisting of accessory strands of nucleic acid, and when necessary, principal strands of nucleic acid).
(3) Hybridizing accessory strands of nucleic acid to the principal strands of nucleic acid to prepare branching nucleic acid strand precursors, hybridizing strands of single-stranded nucleic acid for probes with the branching nucleic acid strand precursors to prepare branching nucleic acid strands, and securing these branching nucleic acid strands to the substrate surface.

The principal strands of nucleic acid, accessory strands of nucleic acid, and strands of single-stranded nucleic acid for probes can all be obtained by known methods. For example, they may be obtained by known methods of synthesizing nucleic acid and by cutting from natural organisms.

### [Method of testing complementarity]

The complementarity test method of the present invention comprises bringing a target nucleic acid into contact with a surface upon which the branching nucleic acid strands of the substrate of the present invention have been secured and testing for complementarity between the target nucleic acid and the probe areas of the branching nucleic acid strand (the probe areas may be areas derived from nucleic acid for probe, the principal strand of nucleic acid, and/or accessory strands of nucleic acid). More specifically, complementarity is tested by detecting by a suitable method the presence of target acid that has hybridized with the probe areas.

Hybridization of the target nucleic acid with a probe area can be detected by suitably employing known methods. Examples of detection methods are the surface plasmon resonance and crystal oscillator methods.

In the surface plasmon resonance method, a laser beam is directed onto the substrate surface and surface plasmon resonance produced on the substrate surface is measured to determine the thickness of the film present on the substrate surface. The presence of nucleic acid strands that have hybridized with the target nucleic acid is identified by differences in film thickness to detect whether hybridization has occurred.

In the surface plasmon resonance method, it is unnecessary to label either the target nucleic acid or nucleic acid strands secured to the substrate, permitting convenient determination of hybridization.

The crystal oscillator method is a method of determining the mass of adhered material from a decrease in frequency due to the adhesion of substances to a crystal oscillator electrode (for example, see Chem. Rev., 1992, 92, 1355-1379).

It is also possible to detect hybridization of a target nucleic acid with a probe area by, for example, employing florescent-labeled DNA as the target nucleic acid and detecting hybridization with the probe area based on fluorescence. Fluorescent-labeled DNA and methods of determining hybridization based on fluorescence are known; these known techniques may be employed without modification in the present invention.

The complementarity testing method of the present invention also permits the detection of target nucleic acid comprising mismatched nucleic acids. That is, in the complementarity testing method of the present invention, there is hybridization with fully complementary target nucleic acid, but there is not hybridization with target nucleic acid containing a single mismatched nucleic acid. Thus, it is possible to detect target acids with single mismatched nucleic acids.

In the complementarity test method of the present invention, a target nucleic acid is desirably brought into contact with the substrate surface in the presence of a divalent metal ion to enhance the stability of the double-stranded portion following hybridization. Examples of divalent metal ions are: magnesium ions, calcium ions, cobalt ions, barium ions, strontium ions, cadmium ions, zinc ions, and iron ions. The concentration of these divalent metal ions suitably falls within a range of from 1 to 1,000 mM.

### Embodiments

Embodiments of the present invention are described below.

### Embodiment 1

Four DNA oligomers were prepared.
20merSH:
5'-ATgCATgCATTAgCATgCTA-3' (SEQ. ID NO. 1)
5-terminal thiolation
80merP:
5'-TggAgAACTgATCgACACAgTTTTTAgAggggTCAAgAggTTTTTAgAggggTCAAgAggTA gCATgCTAATgCATgCAT-3' (SEQ. ID NO. 2)

(From the 5'-terminal, 20 nucleic acids constituted a probe area, 5 nucleic acids (TTTTT) were a spacer, 15 nucleic acids constituted a hybridization area with a first probe of single-stranded nucleic acid, 5 nucleic acids (TTTTT) constituted a spacer, 15 nucleic acids constituted a hybridization area with a second probe of single-stranded nucleic acid, and 20 nucleic acids constituted a hybridization area with 20merSH.)
40merP:
5-CCTCTTgACCCCTCTTTTTTTggAgAACTgATCgACACAg-3' (SEQ. ID NO. 3)
(From the 5'-terminal, 15 nucleic acids constituted a hybridization area with 80merP, 5 nucleic acids (TTTTT) constituted a spacer, and 20 nucleic acids constituted a probe area.)
20merM:
5'-CTgTgTCgATCAgTTCTCCA-3' (SEQ. ID NO. 4)

### [Synthesis of thiolated DNA oligomer]

5'-Terminal thiolated 20merSH was synthesized by the known C6 synthesis method. (For example, see Chemical and Biology Experimental Line 22, Mineo NIWA, "The Chemical Synthesis of DNA", pp. 38-43, Hirokawa Shoten.)

### [Method of preparing probe DNA (branching DNA)]

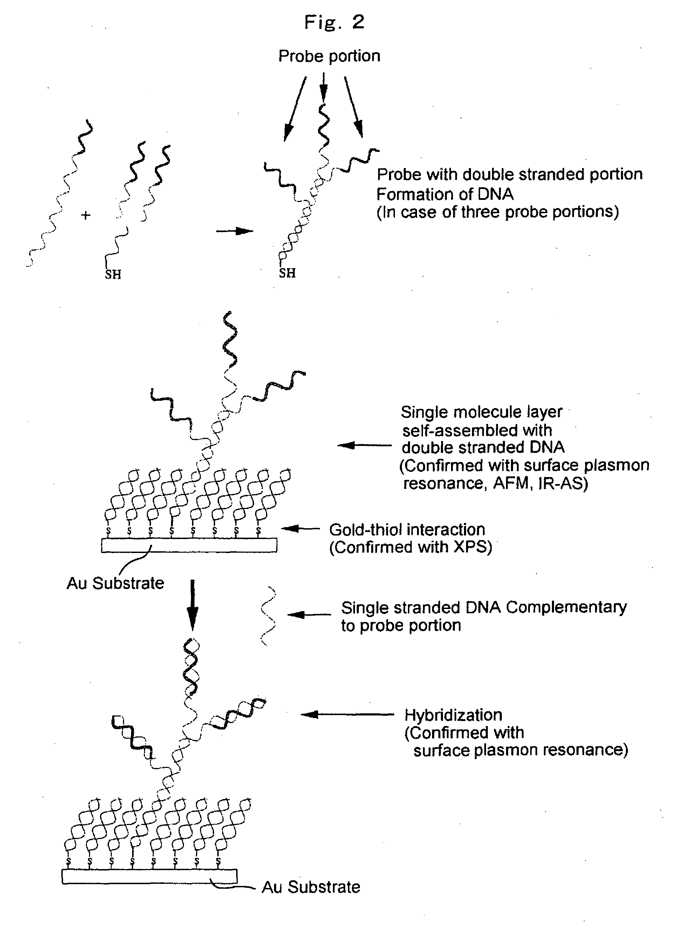

The above-described 80merP, 40merP, and 20merSH were dissolved in buffer solution in a ratio of 1:2:1. This solution was superheated to 95°C and then gradually cooled to room temperature to induce partial hybridization. Ethanol was then added to precipitate the DNA. The DNA obtained was freeze dried to remove the solvent, yielding the probe DNA (branching DNA) shown in Fig. 2. The structure of the DNA obtained was confirmed by electrophoresis.

### [Securing the thiolated double-stranded DNA oligomer to a metal surface]

Employing the same procedure as above, 20mer/20merSH complex of 20mer and 20merSH was formed and employed as a mixed and diluted compound.

As shown in Fig. 2, using the above-described probe DNA (branching DNA) and 20mer/20merSH complex, thiolated double-stranded DNA oligomer was secured to the surface of a gold substrate under the following conditions.

Buffer: MgCl₂(H₂O)₆ was adjusted to a concentration of 20 mM. This solution was then sterilized for 20 min at 120°C and employed as solvent.

Concentration of thiolated double-stranded DNA oligomer: 1.30 D (about 3.0 micromoles).
Temperature: 20°C
Post-treatment: Rinsed with solvent to wash away excess DNA.

### [Hybridization test]

Target DNA was hybridized with a substrate upon which the probe DNA (branching DNA) obtained as set forth above had been secured.
5'-CTGTGTCGATCAGTTCTCCA-3' (20merM) (sequence 4) that was expected to hybridize with the probe site of the probe DNA was employed as target DNA.
5'-CTGTGTCAATCAGTTCTCCA-3' (20merS) (SEQ. ID NO. 5) differing by just one nucleic acid from 20merM was employed as control DNA to test the detection of tautomeric nucleic acid structures (nucleic acid mismatches).
20 mM MgCl₂ aqueous solution was employed as solvent to dissolve the DNA for hybridization. The hybridization temperature was 20°C; hybridization was conducted for two hours.

### [Specific method of evaluating DNA monomolecular film]

Adsorption onto the solid metal substrate and hybridization of target DNA with probe DNA secured on the substrate were observed *in situ* by surface plasmon resonance.

Further, the DNA monomolecular layer was evaluated by atomic force microscopy (AFM), X-ray photoelectron spectroscopy (XPS), and infrared reflection-absorption spectroscopy (IR-RAS).

### [Confirmation of DNA immobilization]

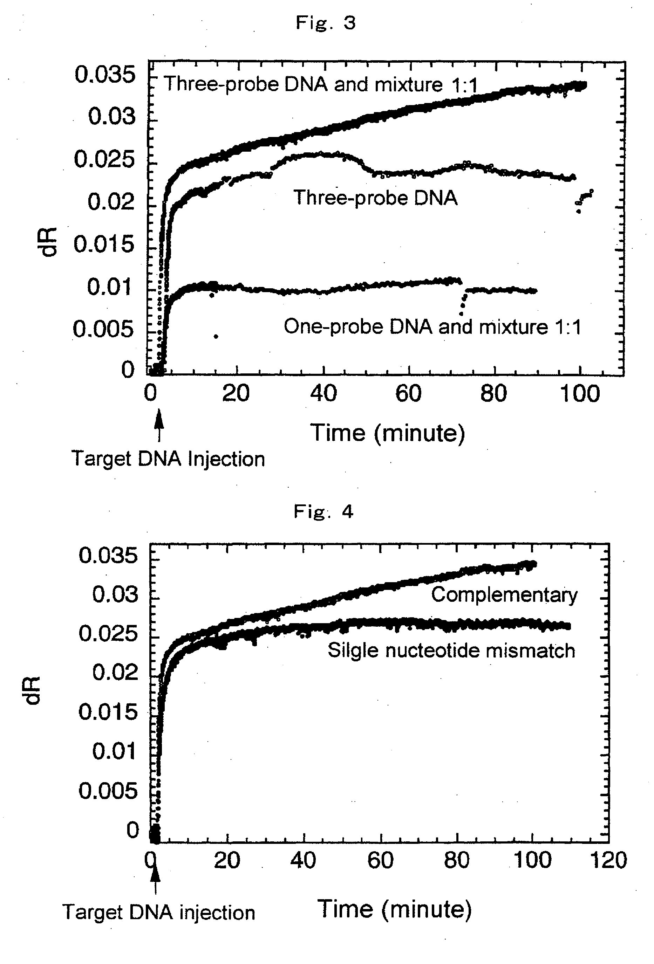

The state of immobilization on a gold substrate surface when the above-described probe DNA (branching DNA) and 20mer/20merSH complex were combined in ratios of 50:50 and 100:0 was observed *in situ* by surface plasmon resonance. The results are given in Fig. 3.

For comparison, 80merP and 20merSH were combined in a 1:1 ratio and dissolved in a buffer solution. The solution was superheated to 95°C and then gradually cooled to room temperature to induce hybridization. Ethanol was then added to precipitate the DNA. The DNA obtained was freeze dried to remove the solvent. The probe DNA complex obtained was secured on the surface of a gold substrate surface in a 50:50 ratio with 20mer/20merSH complex and observed *in situ* by surface plasmon resonance. The results are given in Fig. 3.

When probe DNA (branching DNA) was employed, the intensity of surface plasmon resonance increased about three-fold. It was thus confirmed that securing the probe to the branches increased the amount of target DNA that hybridized.

### [Confirmation of single nucleic acid mismatches]

A substrate with a gold surface upon which the above described complex of a 50:50 ratio of probe DNA (branching DNA) and 20mer/20merSH complex had been secured was employed. The state of hybridization with this substrate of target DNA 5'-CTGTGTCGATCAGTTCTCCA-3' (20merM) (SEQ. ID NO. 4) and control DNA 5'-CTGTGTCAATCAGTTCTCCA-3' (20merS) (SEQ. ID NO. 5) differing in sequence by just one nucleic acid was observed *in situ* by surface plasmon resonance. The results are given in Fig. 4. The results of Fig. 4 reveal that the method of the present invention was capable of identifying a single-nucleic acid mismatch.

### Industrial Applicability

The present invention provides a hybridization substrate in which DNA strands are secured to the surface of a substrate in a manner affording a high surface coverage rate and high activity; a method of manufacturing the same; and a complementarity test method employing this substrate.

## Claims

1. A hybridization substrate having on a substrate surface thereof a branching nucleic acid strand comprising a principal strand of nucleic acid at least one portion of which is attached to the surface of the substrate and at least one portion of which is single-stranded, and at least one strand of single-stranded nucleic acid used as a probe that is partially hybridized to at least one portion of the single-stranded portion of the principal nucleic acid, wherein the principal strand of nucleic acid comprises a portion that is attached to the substrate surface and comprises an end that is free of the substrate surface, with the portion attached to the substrate surface being double-stranded and the portion that is free of the substrate surface being single-stranded.

2. The hybridization substrate according to claim 1 wherein the single-stranded portion of the principal strand of nucleic acid has at least one site in the form of a sequence for hybridizing the single-stranded nucleic acid used as the probe.

3. The hybridization substrate according to any of claims 1 -2 wherein the single-stranded portion of the principal strand of nucleic acid has a probe area within, or on the end of, the portion that is free of the substrate surface.

4. The hybridization substrate according to any of claims 1 to 3 wherein the single-stranded nucleic acid used as the probe has at least a probe area and an area for hybridizing with the single-stranded portion of the principal strand of nucleic acid.

5. A hybridization substrate having on a substrate surface thereof a branching nucleic acid strand comprising:
- a principal strand of nucleic acid at least one portion of which is attached to the surface of the substrate and at least one portion of which is single-stranded;
- at least one accessory strand of nucleic acid that is single-stranded and partially hybridized to at least a portion of the single-stranded portion of the principal strand of nucleic acid;
- at least one strand of single-stranded nucleic acid used as a probe partially hybridized to at least one portion of the single-stranded portion of the principal strand of nucleic acid;
- and/or at least one strand of single-stranded nucleic acid used as a probe partially hybridized to at least a portion of the accessory strand of nucleic acid.

6. The hybridization substrate according to claim 5 wherein the principal strand of nucleic acid comprises a portion that is attached to the substrate surface and has an end that is free of the substrate surface, with the portion attached to the substrate surface being double-stranded and the portion that is free of the substrate surface being single-stranded.

7. The hybridization substrate according to claim 5 or 6 wherein the single-stranded portion of the principal strand of nucleic acid and/or the accessory strand of nucleic acid has at least one site in the form of a sequence for hybridizing a strand of single-stranded nucleic acid used as the probe.

8. The hybridization substrate according to any of claims 5 to 7 wherein the single-stranded portion of the principal strand of nucleic acid has a probe area within, or on the end of, the portion that is free of the substrate surface.

9. The hybridization substrate according to any of claims 5 to 8 wherein the accessory strand of nucleic acid has a probe area on the end of a portion not hybridizing with the single-stranded portion of the principal strand of nucleic acid.

10. The hybridization substrate according to any of claims 5 to 9 wherein the accessory strand of nucleic acid has one site in the form of a sequence for hybridizing with the single-stranded portion of the principal strand of nucleic acid and at least one site in the form of a sequence for hybridizing with the single-stranded nucleic acid used as the probe.

11. The hybridization substrate according to any of claims 1 to 10 wherein the single-stranded nucleic acid used as the probe has at least a probe area and an area for hybridization with the single-stranded portion of the principal strand of nucleic acid and/or the accessory strand of nucleic acid.

12. The hybridization substrate according to claim 11 wherein the single-stranded nucleic acid used as the probe has a spacer area between the probe area and the hybridization area.

13. The hybridization substrate according to any of claims 1 to 12 wherein the branching nucleic acid strand has multiple probe areas, with the nucleic acid sequence of the multiple probe areas being identical.

14. The hybridization substrate according to any of claims 1 to 12 wherein the branching nucleic acid strand has multiple probe areas, with the nucleic acid sequence of the multiple probe areas being different.

15. The hybridization substrate according to any of claims 1 to 14 wherein there are multiple spots formed from multiple branching nucleic acid strands.

16. A method comprising bringing target nucleic acid into contact with a surface upon which has been immobilized the branching nucleic acid strand of the substrate according to any of claims 1 to 15 to test the complementarity of the target nucleic acid with the probe area of the branching nucleic acid strand.

17. The method according to claim 16 wherein the contact of the target nucleic acid with the surface upon which is immobilized the branching nucleic acid strand is conducted in the presence of a divalent metal ion.

18. The method according to claim 17 wherein the divalent metal ion is a magnesium ion.

19. The method according to any of claims 16 to 18 wherein hybridization of the target nucleic acid and the probe area of the branching nucleic acid strand is detected by the surface plasmon resonance method or the crystal oscillator method.

20. The method according of any of claims 16 to 18 wherein the target nucleic acid is labeled with a fluorescent label and hybridization with the probe area of the branching nucleic acid strand is detected by fluorescence.

21. The method according to any of claims 16 to 20 for detecting target nucleic acid containing mismatched nucleic acids.

## Patentansprüche

1. Hybridisierungs-Substrat, aufweisend auf einer Substratoberfläche davon einen sich verzweigenden Nukleinsäure-Strang, der einen Haupt-Strang einer Nukleinsäure, von dem wenigstens ein Bereich an die Oberfläche des Substrats gebunden ist und wenigstens ein Bereich einsträngig ist, und wenigstens einen Strang einer einsträngigen Nukleinsäure umfasst, die als Sonde verwendet wird, die partiell an wenigstens einen Bereich des einsträngigen Bereichs der Haupt-Nukleinsäure hybridisiert ist, worin der Hauptstrang einer Nukleinsäure einen Bereich umfasst, der an die Substratoberfläche gebunden ist, und ein Ende umfasst, das frei von der Substratoberfläche ist, wobei der Bereich, der an die Substratoberfläche gebunden ist, doppelsträngig ist und der Bereich, der frei von der Substratoberfläche ist, einsträngig ist.

2. Hybridisierungs-Substrat nach Anspruch 1, worin der einsträngige Bereich des Hauptstrangs der Nukleinsäure wenigstens eine Site in Form einer Sequenz zum Hybridisieren der einsträngigen Nukleinsäure aufweist, die als Sonde verwendet wird.

3. Hybridisierungs-Substrat nach irgendeinem der Ansprüche 1 bis 2, worin der einsträngige Abschnitt des Hauptstrangs der Nukleinsäure einen Sonden-Bereich innerhalb des Bereichs oder am Ende des Bereichs aufweist, der frei von der Substratoberfläche ist.

4. Hybridisierungs-Substrat nach irgendeinem der Ansprüche 1 bis 3, worin die einsträngige Nukleinsäure, die als Sonde verwendet wird, wenigstens einen Sondenbereich und einen Bereich zum Hybridisieren mit dem einsträngigen Bereich des Hauptstrangs der Nukleinsäure aufweist.

5. Hybridisierungs-Substrat, aufweisend auf einer Substratoberfläche davon einen sich verzweigende Nukleinsäurestrang, der umfasst:
- einen Hauptstrang einer Nukleinsäure, von der wenigstens ein Bereich an die Oberfläche des Substrats gebunden ist und von dem wenigstens ein Bereich einsträngig ist;
- wenigstens einen accessorischen Strang einer Nukleinsäure, die einsträngig ist und partiell an wenigstens einen Bereich des einsträngigen Bereichs des Hauptstrangs der Nukleinsäure hybridisiert ist;
- wenigstens einen Strang einer einsträngigen Nukleinsäure, die als Sonde verwendet wird, die partiell an wenigstens einen Bereich des einsträngigen Bereichs des Hauptstrangs der Nukleinsäure hybridisiert ist;
- und/oder wenigstens einen Strang einer einsträngigen Nukleinsäure, die als Sonde verwendet wird, die partiell an wenigstens einen Bereich des accessorischen Strangs der Nukleinsäure hybridisiert ist.

6. Hybridisierungs-Substrat nach Anspruch 5, worin der Hauptstrang der Nukleinsäure einen Bereich umfasst, der an die Substratoberfläche gebunden ist und ein Ende aufweist, das frei von der Substratoberfläche ist, wobei der Bereich, der an die Substratoberfläche gebunden ist, doppelsträngig ist, und der Bereich, der frei von der Substratoberfläche ist, einsträngig ist.

7. Hybridisierungs-Substrat nach Anspruch 5 oder 6, worin der einsträngige Abschnitt des Hauptstrangs der Nukleinsäure und/oder der accessorische Strang der Nukleinsäure wenigstens eine Site in Form einer Sequenz zum Hybridisieren eines Strangs von einsträngiger Nukleinsäure aufweist, die als Sonde verwendet wird.

8. Hybridisierungs-Substrat nach irgendeinem der Ansprüche 5 bis 7, worin der einsträngige Bereich des Hauptstrangs der Nukleinsäure einen Sondenbereich innerhalb des Bereichs oder am Ende des Bereichs hat, der frei von der Substratoberfläche ist.

9. Hybridisierungs-Substrat nach irgendeinem der Ansprüche 5 bis 8, worin der accessorische Strang von Nukleinsäure einen Sondenbereich am Ende eines Bereichs hat, der nicht mit dem einsträngigen Bereich des Hauptstrangs der Nukleinsäure hybridisiert.

10. Hybridisierungs-Substrat nach irgendeinem der Ansprüche 5 bis 9, worin der accessorische Strang der Nukleinsäure eine Site in Form einer Sequenz zum Hybridisieren mit dem einsträngigen Bereich des Hauptstrangs der Nukleinsäure und wenigstens eine Site in Form einer Sequenz zum Hybridisieren mit der einsträngigen Nukleinsäure aufweist, die als Sonde verwendet wird.

11. Hybridisierungs-Substrat nach irgendeinem der Ansprüche 1 bis 10, worin die einsträngige Nukleinsäure, die als Sonde verwendet wird, wenigstens einen Sondenbereich und einen Bereich zum Hybridisieren mit dem einsträngigen Bereich des Hauptstrangs der Nukleinsäure und/oder dem accessorischen Strang der Nukleinsäure aufweist.

12. Hybridisierungs-Substrat nach Anspruch 11, worin die einsträngige Nukleinsäure, die als Sonde verwendet wird, einen Spacer-Bereich zwischen dem Sondenbereich und dem Hybridisierungs-Bereich aufweist.

13. Hybridisierungs-Substrat nach irgendeinem der Ansprüche 1 bis 12, worin der sich verzweigende Nukleinsäure-Strang mehrere Sonden-Bereiche aufweist, wobei die Nukleinsäure-Sequenz der mehreren Sonden-Bereiche identisch ist.

14. Hybridisierungs-Substrat nach irgendeinem der Ansprüche 1 bis 12, worin der sich verzweigende Nukleinsäure-Strang mehrere Sonden-Bereiche aufweist, wobei die Nukleinsäure-Sequenz der mehreren Sonden-Bereiche unterschiedlich ist.

15. Hybridisierungs-Substrat nach irgendeinem der Ansprüche 1 bis 14, worin es mehrere Spots gibt, die aus mehreren sich verzweigenden Nukleinsäure-Strängen gebildet sind.

16. Verfahren, umfassend ein In-Kontakt-Bringen von Ziel-Nukleinsäure mit einer Oberfläche, auf der der sich verzweigende Nukleinsäure-Strang des Substrats gemäß irgendeinem der Ansprüche 1 bis 15 immobilisiert wurde, zum Testen der Komplimentarität der Ziel-Nukleinsäure mit dem Sonden-Bereich des sich verzweigenden Nukleinsäure-Strangs.

17. Verfahren nach Anspruch 16, worin der Kontakt der Ziel-Nukleinsäure mit der Oberfläche, auf der der sich verzweigende Nukleinsäure-Strang immobilisiert wurde, in Gegenwart eines zweiwertigen Metall-Ions durchgeführt wird.

18. Verfahren nach Anspruch 17, worin das zweiwertige Metall-Ion ein Magnesium-Ion ist.

19. Verfahren nach irgendeinem der Ansprüche 16 bis 18, worin ein Hybridisieren der Ziel-Nukleinsäure und des Sonden-Bereichs des sich verzweigenden Nukleinsäure-Strangs nachgewiesen wird durch das Oberflächen-Plasmon-Resonanz-Verfahren oder das Kristall-Oszillator-Verfahren.

20. Verfahren nach irgendeinem der Ansprüche 16 bis 18, worin die Ziel-Nukleinsäure mit einer fluoreszenten Markierung markiert ist und ein Hybridisieren mit dem Sonden-Bereich des sich verzweigenden Nukleinsäure-Strangs durch Fluoreszenz nachgewiesen wird.

21. Verfahren nach irgendeinem der Ansprüche 16 bis 20 zum Nachweis von Ziel-Nukleinsäuren, die fehlangepasste Nukleinsäuren enthalten.

## Revendications

1. Substrat d'hybridation ayant, sur une surface de substrat de celui-ci, un brin d'acide nucléique ramifié comportant un brin principal d'acide nucléique dont au moins une portion est attachée à la surface du substrat et dont au moins une portion est à l'état simple brin, et au moins un brin d'acide nucléique à l'état simple brin utilisé comme sonde qui s'est partiellement hybridé avec au moins une portion de la portion simple brin de l'acide nucléique principal, dans lequel le brin principal d'acide nucléique comporte une portion qui est attachée à la surface du substrat et comporte une extrémité qui est libre au regard de la surface du substrat, la portion attachée à la surface du substrat étant à l'état double brin, et la portion qui est libre au regard de la surface du substrat étant à l'état simple brin.

2. Substrat d'hybridation selon la revendication 1, dans lequel la portion simple brin du brin principal d'acide nucléique possède au moins un site sous la forme d'une séquence destinée à s'hybrider avec l'acide nucléique à l'état simple brin utilisé comme sonde.

3. Substrat d'hybridation selon l'une quelconque des revendications 1 et 2, dans lequel la portion simple brin du brin principal d'acide nucléique possède un domaine de sonde dans, ou sur l'extrémité de, la portion qui est libre au regard de la surface du substrat.

4. Substrat d'hybridation selon l'une quelconque des revendications 1 à 3, dans lequel l'acide nucléique à l'état simple brin utilisé comme sonde possède au moins un domaine de sonde et un domaine pour s'hybrider avec la portion simple brin du brin principal d'acide nucléique.

5. Substrat d'hybridation ayant, sur une surface de substrat de celui-ci, un brin d'acide nucléique ramifié comportant :
- un brin principal d'acide nucléique dont au moins une portion est attachée à la surface du substrat et au moins une portion est à l'état simple brin,
- au moins un brin annexe d'acide nucléique qui est à l'état simple brin et partiellement hybridé avec au moins une portion de la portion simple brin du brin principal d'acide nucléique,
- au moins un brin d'acide nucléique à l'état simple brin utilisé comme sonde partiellement hybridé avec au moins une portion de la portion simple brin du brin principal d'acide nucléique,
- et/ou au moins un brin d'acide nucléique à l'état simple brin utilisé comme sonde partiellement hybridé avec au moins une portion du brin annexe d'acide nucléique.

6. Substrat d'hybridation selon la revendication 5, dans lequel le brin principal d'acide nucléique comporte une portion qui est attachée à la surface du substrat et possède une extrémité qui est libre au regard de la surface du substrat, la portion attachée à la surface du substrat étant à l'état double brin et la portion qui est libre au regard de la surface du substrat étant à l'état simple brin.

7. Substrat d'hybridation selon la revendication 5 ou 6, dans lequel la portion simple brin du brin principal d'acide nucléique et/ou le brin annexe d'acide nucléique possède au moins un site sous la forme d'une séquence destinée à s'hybrider avec un brin d'acide nucléique à l'état simple brin utilisé comme sonde.

8. Substrat d'hybridation selon l'une quelconque des revendications 5 à 7, dans lequel la portion simple brin du brin principal d'acide nucléique possède un domaine de sonde dans, ou sur l'extrémité de, la portion qui est libre au regard de la surface du substrat.

9. Substrat d'hybridation selon l'une quelconque des revendications 5 à 8, dans lequel le brin annexe d'acide nucléique possède un domaine de sonde sur l'extrémité d'une portion non hybridée avec la portion simple brin du brin principal d'acide nucléique.

10. Substrat d'hybridation selon l'une quelconque des revendications 5 à 9, dans lequel le brin annexe d'acide nucléique possède un site sous la forme d'une séquence destinée à s'hybrider avec la portion simple brin du brin principal d'acide nucléique et au moins un site sous la forme d'une séquence destinée à s'hybrider avec l'acide nucléique à l'état simple brin utilisé comme sonde.

11. Substrat d'hybridation selon l'une quelconque des revendications 1 à 10, dans lequel l'acide nucléique à l'état simple brin utilisé comme sonde possède au moins un domaine de sonde et un domaine d'hybridation avec la portion simple brin du brin principal d'acide nucléique et/ou le brin annexe d'acide nucléique.

12. Substrat d'hybridation selon la revendication 11, dans lequel l'acide nucléique à l'état simple brin utilisé comme sonde possède un domaine d'espacement entre le domaine de sonde et le domaine d'hybridation.

13. Substrat d'hybridation selon l'une quelconque des revendications 1 à 12, dans lequel le brin d'acide nucléique ramifié possède de multiples domaines de sonde, les séquences d'acide nucléique des multiples domaines de sonde étant identiques.

14. Substrat d'hybridation selon l'une quelconque des revendications 1 à 12, dans lequel le brin d'acide nucléique ramifié possède de multiples domaines de sonde, les séquences d'acide nucléique des multiples domaines de sonde étant différentes.

15. Substrat d'hybridation selon l'une quelconque des revendications 1 à 14, dans lequel de multiples spots sont formés à partir de multiples brins d'acide nucléique ramifiés.

16. Procédé comportant l'étape consistant à mettre un acide nucléique ciblé en contact avec une surface sur laquelle a été immobilisé le brin d'acide nucléique ramifié du substrat selon l'une quelconque des revendications 1 à 15 afin de tester la complémentarité de l'acide nucléique ciblé avec le domaine de sonde du brin d'acide nucléique ramifié.

17. Procédé selon la revendication 16, dans lequel la mise en contact de l'acide nucléique ciblé avec la surface sur laquelle est immobilisé le brin d'acide nucléique ramifié est réalisée en présence d'un ion métallique divalent.

18. Procédé selon la revendication 17, dans lequel l'ion métallique divalent est un ion magnésium.

19. Procédé selon l'une quelconque des revendications 16 à 18, dans lequel l'hybridation de l'acide nucléique ciblé et du domaine de sonde du brin d'acide nucléique ramifié est détectée par le procédé de résonance plasmonique de surface ou le procédé à oscillateur à cristal.

20. Procédé selon l'une quelconque des revendications 16 à 18, dans lequel l'acide nucléique ciblé est marqué avec un marqueur fluorescent et l'hybridation avec le domaine de sonde du brin d'acide nucléique ramifié est détectée par fluorescence.

21. Procédé selon l'une quelconque des revendications 16 à 20 pour détecter un acide nucléique ciblé contenant des acides nucléiques mésappariés.


### SEQUENCE LISTING

<110> RIKEN
<120> Substrate for hybridization and the use thereof
<130> A35043H
<160> 5
<210> 1
   <211> 20
   <212> DNA
   <213> Artificial Sequence
<400> 1
   atgcatgcat tagcatgcta 20
<210> 2
   <211> 80
   <212> DNA
   <213> Artificial Sequence
<400> 2
   tggagaactg atcgacacag tttttagagg ggtcaagagg tttttagagg ggtcaagagg 60
   tagcatgcta atgcatgcat 80
<210> 3
   <211> 40
   <212> DNA
   <213> Artificial Sequence
<400> 3
   cctcttgacc cctctttttt tggagaactgatcgacacag 40
<210> 4
   <211> 20
   <212> DNA
   <213> Artificial Sequence
<400> 4
   ctgtgtcgat cagttctcca 20
<210> 5
   <211> 20
   <212> DNA
   <213> Artificial Sequence
   <400> 5
   ctgtgtcaat cagttctcca 20

### SEQUENCE LISTING

<110> RIKEN
   <120> Substrate for hybridization and the use thereof
   <130> A35043H
<160> 5
<210> 1
   <211> 20
   <212> DNA
   <213> Artificial Sequence
<400> 1
   atgcatgcat tagcatgcta 20
<210> 2
   <211> 80
   <212> DNA
   <213> Artificial Sequence
<400> 2
   tggagaactg atcgacacag tttttagagg ggtcaagagg tttttagagg ggtcaagagg 60
   tagcatgcta atgcatgcat 80
<210> 3
   <211> 40
   <212> DNA
   <213> Artificial Sequence
<400> 3
   cctcttgacc cctctttttt tggagaactgatcgacacag 40
<210> 4
   <211> 20
   <212> DNA
   <213> Artificial Sequence
<400> 4
   ctgtgtcgat cagttctcca 20
<210> 5
   <211> 20
   <212> DNA
   <213> Artificial Sequence
<400> 5
   ctgtgtcaat cagttctcca 20